# EUROPEAN PATENT SPECIFICATION

(11) **EP 3 781 134 B1**
(45) Date of publication and mention of the grant of the patent: **22.11.2023**
(21) Application number: 19725227.3
(22) Date of filing: 19.04.2019
(51) Int. Cl.: A61K 9/20, A61K 9/28, A61K 9/48, A61K 9/50, C12N 1/20

(54) **ORAL COMPOSITION FOR USE AS ENHANCER OF EUBIOTIC BACTERIA ALREADY PRESENT AT THE INTESTINAL LEVEL**
ORALE ZUSAMMENSETZUNG ZUR VERWENDUNG ALS VERSTÄRKER VON EUBIOTISCHEN BAKTERIEN, DIE BEREITS AUF DER INTESTINALEN EBENE VORHANDEN SIND
COMPOSITION ORALE DESTINÉE À ÊTRE UTILISÉE EN TANT QU'ACTIVATEUR DE BACTÉRIES EUBIOTIQUES DÉJÀ PRÉSENTES AU NIVEAU INTESTINAL

(30) Priority: 20.04.2018 IT 201800004764
(43) Date of publication of application: 24.02.2021
(73) Proprietor: Labomar S.p.A., 31036 Istrana (TV) (IT)
(72) Inventor: BERTIN, Walter, 31036 Istrana TV (IT); FRATTER, Andrea, 31036 Istrana TV (IT)
(74) Representative: Perani & Partners S.p.A.
(86) International application number: PCT/IB2019/053253
(87) International publication number: WO 2019/202559

(56) References cited:
- US-A1- 2016 022 592
- HASSAN PYAR ET AL: "Abstract", ACTA PHARMACEUTICA, vol. 64, no. 2, 1 June 2014 (2014-06-01), pages 247-256, XP055534032, HR ISSN: 1330-0075, DOI: 10.2478/acph-2014-0011
- U.S. Dairy Export Council: "Nutritional composition of skim milk powder", , 1 January 2005 (2005-01-01), page 41, XP055534136, Retrieved from the Internet: URL:https://www.google.com/url?sa=t&rct=j& q=&esrc=s&source=web&cd=1&ved=2ahUKEwj2iIO 875zfAhUPzqQKHTmSBTsQFjAAegQIABAC&url=http s%3A%2F%2Fwww.dairyglobalnutrition.org%2FD ocuments%2FDairy%2520Nutrition%2FNutrCompO fSMP.pdf&usg=AOvVaw3Tq84-JHKEYGbmlkDkJ-eT [retrieved on 2018-12-13]

## Description

### FIELD OF THE INVENTION

The present invention relates to oral compositions capable of enhancing the proliferation and growth of eubiotic bacteria already present at the intestinal level, with said compositions and the use of the same for the treatment of disorders caused by the reduced presence of eubiotic bacteria.

### STATE OF THE ART

The state of the art of integration with probiotics and prebiotics generally involves the association of one or more bacterial strains, such as lactobacilli and bifids, as capsules or anhydrous sachets and preferably in association with so-called prebiotic substances, namely substances capable of nourishing bacterial strains. Despite the undoubted effectiveness of probiotic therapies, which find application in various pathological conditions or alteration of enteric eubiosis, the integration of prebiotic fibres such as inulin, FOS, GOS at dosages often very variable and without certainty of their transmission in the correct enteric district cannot be considered as effective as the simultaneous intake of probiotics. There is in fact no certainty of the correct dislocation of said soluble fibres in the intestinal tract showing a greater presence of bacteria upon oral intake. Not even a partial absorption can be excluded. These fibres can also, upon bacteria fermentation, produce intestinal gas and create abdominal discomfort, swelling up to cramps.

Hassan Pyar et al "Abstract" ACTA PHARMACEUTICA, vol.64. no. 2, June 1, 2014 (2014-06-01) pages 247-256, XP055534032IIR ISSN:1330-0075, DOI:10.2478/acph-2014-0011, discloses a capsule composition comprising granules coated with an enteric composition based on EU-DRAGIT L, whose granules include lactobacilli, an edible medium constituted by a broth based on peptone (partially hydrolysed protein), magnesium sulphate, glucose, skimmed milk powder (that is known to comprise at least one essential amino acid, together with lactobacilli). The mixture is granulated by adding corn starch (that is a mucoadhesive polysaccharide) and dried in the oven. The previously dried granules obtained are then coated with Eudragit L by spray technique.

### SUMMARY OF THE INVENTION

The Applicant has now found a new combination of substances used as coadjuvants as defined by the claims, for the growth of bacteria in a culture medium (cellular soils), in the form of a tablet, powder or gastro-resistant capsule, capable of creating a gel adherent to the epithelium in the distal part of the intestine through which the above substances can spread and tiotirisli the bacteria, thus generating a real enteric culture substrate. The polymer used for coating the tablet or included in the shell of the capsule of the present invention is an anionic copolymer capable of releasing the active substances at the level of the colon and containing repetitive units of the following monomers:
i) esters of acrylic acid, methacrylic acid with C1-C4, preferably C1-C2 alcohols and relative mixtures of said alcohols;
ii) acrylic acid, methacrylic acid and their mixtures,
iii) the ratio between the carboxyalkyl functional ester groups and the free carboxylic functional groups (COOH) in said polymer is between 8:1 and 12:1.

The aforementioned combination of substances used as coadjuvants in the growth of eubiotic bacteria consists of the following components:
a) at least one essential amino acid,
b) at least one protein hydrolysate of vegetable or animal origin,
c) at least one trace element for use in the food or biochemical field,
d) at least one sugar selected from monosaccharides or di-saccharides,
e) at least one mucoadhesive polysaccharide capable of gelling when in contact with the intestinal mucosa, in a composition as defined by the claims.

This composition is characterized in that that it is free of eubiotic bacteria and in particular probiotics and is able to enhance the proliferation and growth of eubiotic bacteria, in particular probiotics, at the intestinal level.

The eubiotic bacteria, in particular probiotics, present at the intestinal level are endogenous and possibly also exogenous, and these latter are present at the intestinal level upon a separate administration with respect to the oral composition object of the invention.

A further object of the present invention relates to the use of said oral compositions as defined by the claims, for the treatment of disorders caused by the reduced presence of eubiotic bacteria.

### DETAILED DESCRIPTION OF THE INVENTION

For the purposes of the present invention, an essential amino acid means an amino acid, which a vertebrate organism is not able to synthesize in sufficient amounts and which therefore must be taken with food.

To this category belong in particular the following amino acids: L-phenylalanine, L-leucine, L-histidine, L-isoleucine, L-lysine, S-methionine, L-threonine, L-tryptophan, and L-valine.

Preferably, for the purposes of the invention, L-valine, L-leucine, L-isoleucine and relative mixtures are used.

According to a particularly preferred solution, a mixture of the aforementioned three amino acids in a weight ratio of 1:1:1 is used.

For the purposes of the present invention, trace element for use in the food or biochemical field means an inorganic micronutrient, which is taken in trace amounts. For the purposes of the present invention, on the other hand, a micronutrient means a nutritional principle that is necessary for humans and other living beings, which the organisms are unable to produce and which is taken in small amounts.

The inorganic micronutrients are preferably selected from Na, K, Mg, Ca, P and Mn. According to a more preferred solution, component c) is preferably magnesium in the form of a salt, for example magnesium sulphate, magnesium chloride, magnesium pidolate, etc. According to a particularly preferred solution, magnesium sulphate is used in weight ratios of 1:1 with respect to the single amino acid.

For the purposes of the present invention, a protein hydrolysate of animal or vegetable origin means a mixture of more or less complex amino acids and peptides obtained from the hydrolysis of an animal protein source (e.g. whey proteins, egg proteins) or a vegetable protein source (e.g. soy proteins). Preferably, for the purposes of the present invention, casein hydrolysate is used as component b) and even more preferably in concentrations between 2 and 15% on the total weight of said composition.

Sugars or component d) are selected from: glucose, maltose, fructose, sucrose and lactose or mixtures thereof.

According to a particularly preferred solution, sugar is present in the oral composition according to the present invention in a weight ratio ranging from 30 to 60% by weight.

Preferably, the anionic copolymer is a copolymer of methyl acrylate, methyl methacrylate and methacrylic acid in which the ratio between said monomer units is 7:3:1 respectively. Consequently, the ratio between carboxyalkyl groups and free carboxylic functional groups (COOH) in said polymer is 10:1.

This polymer, which has a weight average molecular weight of 280,000 Da measured with GPC, is commercially available under the trade name Eudraguard^{®} biotic.

The compositions object of the present invention contain the components a) - d) dispersed in one or more mucoadhesive polysaccharides e) capable of gelling when in contact with the intestinal mucosa.

Therefore, the oral compositions according to the invention thanks to the presence of the anionic copolymer are released at the level of the colon and, thanks to the presence of one or more gelling and mucoadhesive polysaccharides, the composition in contact with the intestine is transformed into an adhesive gel-matrix that harnesses the bacteria present in that enteric area and puts them in contact with nutrients, thus creating a sort of enteric "Petri plate".

Preferably, these gelling and mucoadhesive polysaccharides are in particular selected from agar agar and sodium alginate and relative mixtures. According to a particularly preferred solution, they are a mixture of these polysaccharides.

For the purposes of the present invention, eubiotic bacteria and in particular endogenous probiotics mean those bacteria already present at the intestinal level, exogenous eubiotic bacteria mean those bacteria that are not already present at the intestinal level, but that will be present upon their administration, in any case separate with respect to the oral composition according to the present invention that, as previously mentioned, is free of such bacteria.

The oral compositions of the present invention as defined by the claims, further contain excipients of the conventional type such as thickeners, lubricants, flavourings, humectants, etc.

The daily and medium-long term intake of said pharmaceutical form guarantees an overall coverage of a large colon intestinal area, since said oral composition, once reached the colon, will adhere to a different even if adjacent point, thus guaranteeing an overall good coverage and an even eubiotic growth action. This strategy allows the growth of the eubiotic strains by modulating the ratios among the above substances in a specific way and avoiding the fermentation phenomena of the prior art formulations due to the massive presence of probiotic sugars (inulin, FOS, GOS). Moreover, this strategy introduces a eubiotic growth approach, which does not necessarily include the exogenous probiotic intake, which would still represent a single small component of the overall eubiotic flora, but includes the proliferative stimulus of those already present at the intestinal level. This approach is therefore particularly suitable in cases of reduced presence of eubiotic bacteria, for example after prolonged antibiotic therapies or incongruous lifestyles and eating habits or after surgery or pathologies on an inflammatory or enteric atrophic basis.

Here below, for illustrative but not limitative purposes, the compositions of three formulations prepared with the same modalities are reported and in the following examples the booster activity of the three formulations with regard to some probiotics that are found at the intestinal level is compared to the booster effect exerted by the prebiotic inulin.

### FORMULATION 1 - COATED TABLET

| ***Raw materia**l* | ***gr*/*CPR*** |
|---|---|
| SUCROSE | 0.66000 |
| CASEIN HYDROLISATES | 0.06000 |
| MAGNESIUM SULPHATE | 0.00360 |
| ISOLEUCINE L | 0.00360 |
| LEUCINE L | 0.00360 |
| VALINE L | 0.00360 |
| POLYSORBATE T80 | 0.01800 |
| SODIUM ALGINATE | 0.05000 |
| AGAR AGAR POWDER | 0.05000 |
| DIHYDRATE DIBASIC CALCIUM PHOSPHATE | 0.21760 |
| PRECIPITATED AMORPHOUS SILICA | 0.01000 |
| MAGNESIUM STEARATE | 0.02000 |
| MICROCRYSTALLINE CELLULOSE | 0.10000 |
| ANIONIC METHACRYLATE COPOLYMER | 0.03982 |
| TRIETHYLCITHRATE | 0.00199 |
| TALC | 0.01991 |
| TOTAL | 1.26172 |

### FORMULATION 2 - COATED TABLET

| **COMPONENT** | **g/cpr** |
|---|---|
| MALTOSE | 0.66000 |
| CASEIN HYDROLISATES | 0.06000 |
| MAGNESIUM SULPHATE | 0.00360 |
| ISOLEUCINE L | 0.00360 |
| LEUCINE L | 0.00360 |
| VALINE L | 0.00360 |
| POLYSORBATE T80 | 0.01800 |
| SODIUM ALGINATE | 0.05000 |
| AGAR AGAR POWDER | 0.05000 |
| DIHYDRATED DIBASIC CALCIUM PHOSPHATE | 0.21760 |
| PRECIPITATED AMORPHOUS SILICA | 0.01000 |
| MAGNESIUM STEARATE | 0.02000 |
| MICROCRYSTALLINE CELLULOSE | 0.10000 |
| ANIONIC METHACRYLATE COPOLYMER | 0.03982 |
| TRIETHYLCITHRATE | 0.00199 |
| TALC | 0.01991 |
| TOTAL | 1.26172 |

### FORMULATION 1 - COATED TABLET

| **COMPONENT** | **g/cpr** |
|---|---|
| FRUCTOSE | 0.66000 |
| CASEIN HYDROLISATES | 0.06000 |
| MAGNESIUM SULPHATE | 0.00360 |
| ISOLEUCINE L | 0.00360 |
| LEUCINE L | 0.00360 |
| VALINE L | 0.00360 |
| POLYSORBATE T80 | 0.01800 |
| SODIUM ALGINATE | 0.05000 |
| AGAR AGAR POWDER | 0.05000 |
| DIHYDRATED DIBASIC CALCIUM PHOSPHATE | 0.21760 |
| PRECIPITATED AMORPHOUS SILICA | 0.01000 |
| MAGNESIUM STEARATE | 0.02000 |
| MICROCRYSTALLINE CELLULOSE | 0.10000 |
| ANIONIC METHACRYLATE COPOLYMER | 0.03982 |
| TRIETHYLCITHRATE | 0.00199 |
| TALC | 0.01991 |
| TOTAL | 1.26172 |

### EXAMPLE OF PREPARATION OF TABLETS WITH THE ABOVE REPORTED FORMULATIONS 1-3

### Method of preparation of the tablet core and compression

a. Dry weight ISOLEUCINE, LEUCINE and VALINE.
b. Moisten the mixture obtained from a. with POLYSORBATE.
c. Dry the mixture from b. with the sugar chosen for each formulation.
d. Add SILICA to the system obtained from c.
e. Sift the mixture obtained from d.
f. Add to the composition obtained from e. CASEIN HYDROLYSATES, DIBASIC DIHYDRATED DIBASIC CALCIUM PHOSPHATE, MAGNESIUM SULPHATE, MICROCRYSTALLINE CELLULOSE, SODIUM ALGINATE, AGAR AGAR and MAGNESIUM STEARATE.
g. Compress the powder obtained from f.

### Method of preparation of the film and filming

h. Separately dilute and place under mild stirring the ANIONIC METHACRYLATE COPOLYMER.
i. Dilute TRIETHYLCYTRATE in a portion of the water provided for the coating.
j. Homogenize the TALC in a portion of the water provided for the coating.
k. Combine the solution obtained from i. to the solution obtained from h.
l. Combine the solution obtained from j. to the solution obtained from k.
m. Film the nuclei of the tablets obtained from g.

### EXAMPLE 2 - BOOSTER ACTIVITY COMPARISON OF FORMULATIONS 1-3 AND INULIN

These tests were conducted according to the following operating method.
1. Hydrate the growth medium with osmotic water and sterilize it at 121°C for 15 minutes;
2. Cool the medium obtained from 1. and place it in a thermostatic bath at 45°C until use;
3. Separately suspend 10 g of lactic acid bacteria in 90 ml of casein soya bean digest broth (tryptone soya. broth TSB) (mother dilution) for about 30/60 seconds using the appropriate homogenizer (stomacher),
4. Prepare a series of successive decimal dilutions from the mother dilution obtained from 3. until reaching the suitable dilution to allow a correct count of the microorganisms;
5. Sow in each Petri plate of 1 ml of the suitable dilution (agar-germ method) obtained from 4,
6. Add to the Petri plate the agar medium (about 15 ml) obtained from 2. and shake the plates circularly and with cross movements to favour a homogeneous distribution of the diluted sample;
7. Wait for the agar to solidify perfectly and at the end place the dishes with the lid facing downwards in the incubator at 37°C ± 1°C for 72 hours;
8. Count the colonies on plates containing less than 300 cfu/g;
9. Calculate the arithmetic mean of the "read" colonies in the two plates having the same dilution and multiply it by the dilution factor.

| | | **(Probiotic booster) TABLET 1** | **(Probiotic booster) TABLET 2** | **(Probiotic booster) TABLET 3** |
|---|---|---|---|---|
| | **UFC/g INULIN** | **UFC/g Powder with saccharose** | **UFC/g Powder with fructose** | **UFC/g Powder with maltose** |
| LACTOBACILLUS ACIDOPHILUS 200 MLD/g LA -14 DANISCO | 2,0 x 10⁵ | 2,8 x 10¹¹ | 8,0 x 10¹⁰ | 2,6 x 10¹¹ |
| LACTOBACILLUS RHAMNOSUS 350 MLD/g LGG Probio-Tec^{®} LGG^{®} | 4,0 x 10⁵ | 2,9 x 10¹¹ | 2,1 x 10¹¹ | 4,5 x 10¹¹ |
| BIFIDUM BACTERIUM LACTIS BI - 04 500 MLD/G | 6,0 x 10⁵ | 6,0 x 10⁶ | 3,0 x 10⁶ | 5,1 x 10⁶ |

### EXAMPLE 3

### Booster activity of tablets 1-3 against additional lactic acid bacteria

| Growths | UFC/g Tablet 1 | UFC/g Tablet 2 |
|---|---|---|
| LACTOBACILLUS CASEI 300 MLD/g LC-11 | 3,7 x 10¹⁰ | 4,1 x 10¹² |

| Growths | UFC/g Tablet 1 | UFC/g Tablet 2 |
|---|---|---|
| BIFIDUM BACTERIUM BIFIDUM (BB-06) 100 MLD/g | 3,1 x 10⁹ | 6,1x 10⁵ |

## Claims

1. Oral colonic release compositions able to enhance the proliferation and growth of both endogenous eubiotic bacteria, in particular probiotics, already present at the intestinal level, and of exogenous eubiotic bacteria present at the intestinal level upon their separate and extemporaneous administration, said compositions being in the form of tablets, powders or gastro-resistant capsules, consisting of:
A) a combination of substances used as co-adjuvants for the growth of eubiotic bacteria, said combination consisting of:
a) at least one essential amino acid,
b) at least one protein hydrolysate of vegetable or animal origin,
c) at least one trace element for use in the food or biochemical fields,
d) at least one sugar selected from monosaccharides or di-saccharides,
e) at least one mucoadhesive polysaccharide, capable of gelling in contact with the intestinal mucosa,
B) conventional excipients
wherein said tablets or powders are coated with, or the outer shell of said capsules comprises, an anionic copolymer, simultaneously containing repeating units of the following monomers:
i) esters of acrylic and methacrylic acid with C1-C4, preferably C1-C2, alcohols and relative mixtures of said alcohols;
ii) acrylic acid, methacrylic acid and relative mixtures,
iii) wherein the ratio between the ester functional groups (carboxyalkyl) and the free carboxylic functional groups (COOH) in said polymer is between 8:1 and 12:1, and is able to release the components a) - e) at the level of the colon,
said oral colonic release compositions being free from eubiotic, in particular probiotic bacteria and being able to enhance proliferation and growth of eubiotic, in particular probiotic bacteria at the intestinal level, wherein said eubiotic, in particular probiotic bacteria are of endogenous and optionally exogenous type, these latter being present at the intestinal level upon a separate administration with respect to said oral colonic release composition.

2. Oral compositions according to claim 1, wherein said at least one essential amino acid is selected among L-valine, L-leucine, L-isoleucine and relative mixtures.

3. Oral compositions according to claim 2, wherein said at least one essential amino acid is a mixture of L-valine, L-leucine, L-isoleucine in a weight ratio of 1:1:1.

4. Oral compositions according to any one of claims 1-3, wherein the inorganic micronutrient is magnesium.

5. Oral compositions according to claim 4, wherein magnesium is present in the form of magnesium sulphate in a weight ratio of 1:1 with respect to the single essential amino acid.

6. Oral compositions according to any one of the claims 1-5, wherein the protein hydrolysate is casein hydrolysate in concentrations ranging from 2 to 15% on the total weight of said composition.

7. Oral compositions according to any one of the claims 1-6, wherein said mucoadhesive polysaccharide capable of gelling in contact with the intestinal mucosa and mucoadhesive is selected among sodium alginate, agar agar or relative mixtures.

8. Oral compositions according to any one of the claims 1-7, wherein the sugar is selected among glucose, sucrose, maltose, fructose, lactose or their mixtures, and is preferably present in concentrations ranging from 30 to 60% by weight on the total weight of said composition.

9. Oral compositions according to any one of claims 1-8, wherein the anionic copolymer is a copolymer of methyl acrylate, methyl methacrylate and methacrylic acid, in which the ratio among said monomer units is respectively 7:3:1 and preferably has a weight average molecular weight of 280,000 Da.

10. Oral compositions according to any one of claims 1 to 9 for use in the treatment of disorders caused by reduced presence of eubiotic bacteria.

11. Compositions for use according to claim 10, wherein said disorders are caused by prolonged antibiotic therapies, incongruous lifestyles and eating habits or occur after surgery or due to inflammatory or atrophic enteric pathologies.

## Patentansprüche

1. Orale Dickdarmfreisetzungszusammensetzungen, die in der Lage sind, die Proliferation und das Wachstum sowohl endogener eubiotischer Bakterien, insbesondere Probiotika, die bereits auf Darmebene vorhanden sind, als auch exogener eubiotischer Bakterien, die auf Darmebene vorhanden sind, bei ihrer getrennten und extemporären Verabreichung zu fördern, wobei die Zusammensetzungen in Form von Tabletten, Pulvern oder magensaftresistenten Kapseln vorliegen, bestehend aus:
A) einer Kombination von Substanzen, die als Co-Adjuvanzien für das Wachstum eubiotischer Bakterien verwendet werden, wobei die Kombination besteht aus:
a) mindestens einer essentiellen Aminosäure,
b) mindestens einem Proteinhydrolysat pflanzlichen oder tierischen Ursprungs,
c) mindestens einem Spurenelement zur Verwendung im Lebensmittel- oder biochemischen Bereich,
d) mindestens einem Zucker ausgewählt aus Monosacchariden oder Disacchariden,
e) mindestens einem mucoadhäsiven Polysaccharid, das in Kontakt mit der Darmschleimhaut gelieren kann,
B) konventionelle Hilfsstoffe
wobei die Tabletten oder Pulver mit einem anionischen Copolymer beschichtet sind oder die äußere Hülle der Kapseln ein anionisches Copolymer umfasst, das gleichzeitig wiederkehrende Einheiten der folgenden Monomere enthält:
i) Ester der Acryl- und Methacrylsäure mit C1-C4-, bevorzugt C1-C2-Alkoholen und entsprechende Mischungen dieser Alkohole;
ii) Acrylsäure, Methacrylsäure und entsprechende Mischungen,
iii) wobei das Verhältnis zwischen den funktionellen Estergruppen (Carboxyalkyl) und den freien unktionellen Carboxylgruppen (COOH) in dem Polymer zwischen 8:1 und 12:1 liegt und in der Lage ist, die Komponenten a) - e) auf der Ebene des Dickdarms freizusetzen,
wobei die oralen Dickdarmfreisetzungszusammensetzungen frei von eubiotischen, insbesondere probiotischen Bakterien sind und in der Lage sind, die Proliferation und das Wachstum eubiotischer, insbesondere probiotischer Bakterien auf Darmebene zu fördern, wobei die eubiotischen, insbesondere probiotischen Bakterien von endogenem und gegebenenfalls exogenem Typ sind, wobei Letztere bei einer separaten Verabreichung in Bezug auf die orale Dickdarmfreisetzungszusammensetzung auf Darmebene vorliegen.

2. Orale Zusammensetzungen nach Anspruch 1, wobei die mindestens eine essentielle Amino
säure ausgewählt ist aus L-Valin, L-Leucin, L-Isoleucin und relativen Mischungen.

3. Orale Zusammensetzungen nach Anspruch 2, wobei die mindestens eine essentielle Amino
säure eine Mischung aus L-Valin, L-Leucin, L-Isoleucin im Gewichtsverhältnis 1:1:1 ist.

4. Orale Zusammensetzung nach einem der Ansprüche 1-3, wobei der anorganische Mikronährstoff Magnesium ist.

5. Orale Zusammensetzungen nach Anspruch 4, wobei Magnesium in der Form von Magnesiumsulfat im Gewichtsverhältnis 1:1 zu der einzelnen essentiellen Aminosäure vorliegt.

6. Orale Zusammensetzungen nach einem der Ansprüche 1-5, wobei das Proteinhydrolysat Caseinhydrolysat in Konzentrationen im Bereich von 2 bis 15 % des Gesamtgewichts der Zusammensetzung ist.

7. Orale Zusammensetzungen nach einem der Ansprüche 1-6, wobei das mucoadhäsive Polysaccharid, das in Kontakt mit der Darmschleimhaut gelieren kann, und das Mucoadhäsiv aus Natriumalginat, Agar-Agar oder relativen Mischungen ausgewählt ist.

8. Orale Zusammensetzungen nach einem der Ansprüche 1-7, wobei der Zucker aus Glucose, Saccharose, Maltose, Fructose, Lactose oder deren Mischungen ausgewählt ist und bevorzugt in Konzentrationen im Bereich von 30 bis 60 Gew.-%, bezogen auf das Gesamtgewicht der Zusammensetzung, vorliegt.

9. Orale Zusammensetzungen nach einem der Ansprüche 1-8, wobei das anionische Copolymer ein Copolymer von Methylacrylat, Methylmethacrylat und Methacrylsäure ist, wobei das Verhältnis zwischen den Monomereinheiten jeweils 7:3: 1 beträgt und bevorzugt ein gewichtsmittleres Molekulargewicht von 280.000 Da aufweist.

10. Orale Zusammensetzungen nach einem der Ansprüche 1 bis 9 zur Verwendung bei der Behandlung von Störungen, die durch verminderte Anwesenheit eubiotischer Bakterien verursacht werden.

11. Zusammensetzungen zur Verwendung nach Anspruch 10, wobei die Störungen durch verlängerte Antibiotika-Therapien, inkongruente Lebens- und Essgewohnheiten verursacht werden oder nach einer Operation oder aufgrund von entzündlichen oder atrophischen enterischen Pathologien auftreten.

## Revendications

1. Compositions orales à libération colique capables d'améliorer la prolifération et la croissance à la fois des bactéries eubiotiques endogènes, en particulier des probiotiques, déjà présentes au niveau intestinal, et des bactéries eubiotiques exogènes présentes au niveau intestinal lors de leur administration séparée et extemporanée, lesdites compositions se présentant sous forme de comprimés, de poudres ou de capsules gastro-résistantes, constituées de :
A) une combinaison de substances utilisées comme co-adjuvants pour la croissance des bactéries eubiotiques, ladite combinaison étant constitué de :
a) au moins un acide aminé essentiel,
b) au moins un hydrolysat de protéines d'origine végétale ou animale,
c) au moins un oligo-élément destiné à être utilisé dans les domaines alimentaire ou biochimique,
d) au moins un sucre sélectionné parmi les monosaccharides ou les disaccharides,
e) au moins un polysaccharide mucoadhésif, capable de se gélifier au contact de la muqueuse intestinale,
B) excipients classiques
dans lesquelles lesdits comprimés ou poudres sont enrobés de, ou l'enveloppe extérieure desdites capsules comprend, un copolymère anionique, contenant simultanément des unités répétitives des monomères suivants :
i) les esters de l'acide acrylique et méthacrylique avec des alcools en C1-C4, de préférence en C1-C2, et les mélanges relatifs desdits alcools ;
ii) l'acide acrylique, l'acide méthacrylique et les mélanges relatifs,
iii) dans lequel le rapport entre les groupes fonctionnels ester (carboxyalkyle) et les groupes fonctionnels carboxyliques libres (COOH) dans ledit polymère est compris entre 8:1 et 12: 1, et est capable de libérer les composants a) à e) au niveau du côlon,
lesdites compositions orales à libération colique étant exemptes de bactéries eubiotiques, en particulier probiotiques et étant capables d'améliorer la prolifération et la croissance de bactéries eubiotiques, en particulier de bactéries probiotiques au niveau intestinal, lesdites bactéries eubiotiques, en particulier les bactéries probiotiques, étant de type endogène et éventuellement exogène, ces dernières étant présentes au niveau intestinal lors d'une administration séparée par rapport à ladite composition orale à libération colique.

2. Compositions orales selon la revendication 1, dans lesquelles ledit au moins un acide aminé essentiel
est sélectionné parmi la L-valine, la L-leucine, la L-isoleucine et les mélanges relatifs.

3. Compositions orales selon la revendication 2, dans lesquelles ledit au moins un acide aminé essentiel
est un mélange de L-valine, L-leucine, L-isoleucine dans un rapport pondéral de 1:1:1.

4. Compositions orales selon l'une quelconque des revendications 1 à 3, dans lesquelles le micronutriment inorganique est le magnésium.

5. Compositions orales selon la revendication 4, dans lesquelles le magnésium est présent sous forme
de sulfate de magnésium dans un rapport pondéral de 1:1 par rapport à l'acide aminé essentiel unique.

6. Compositions orales selon l'une quelconque des revendications 1 à 5, dans lesquelles l'hydrolysat de protéines est un hydrolysat de caséine dans des concentrations allant de 2 à 15 % du poids total de ladite composition.

7. Compositions orales selon l'une quelconque des revendications 1 à 6, dans lesquelles ledit polysaccharide mucoadhésif capable de se gélifier au contact de la muqueuse intestinale et mucoadhésive est sélectionné parmi l'alginate de sodium, l'agar-agar ou des mélanges relatifs.

8. Compositions orales selon l'une quelconque des revendications 1 à 7, dans lesquelles le sucre est sélectionné parmi le glucose, le saccharose, le maltose, le fructose, le lactose ou leurs mélanges, et est de préférence présent dans des concentrations allant de 30 à 60 % en poids par rapport au poids total de ladite composition.

9. Compositions orales selon l'une quelconque des revendications 1 à 8, dans lesquelles le copolymère anionique est un copolymère d'acrylate de méthyle, de méthacrylate de méthyle et d'acide méthacrylique, dans lequel le rapport entre lesdites unités de monomères est respectivement de 7:3:1 et a de préférence un poids moléculaire moyen en poids de 280 000 Da.

10. Compositions orales selon l'une quelconque des revendications 1 à 9 destinées à être utilisées dans le traitement de troubles causés par une présence réduite de bactéries eubiotiques.

11. Compositions destinées à être utilisées selon la revendication 10, dans lesquelles lesdits troubles sont causés par des thérapies antibiotiques prolongées, des modes de vie et des habitudes alimentaires incongrus ou surviennent après une intervention chirurgicale ou en raison de maladies entériques inflammatoires ou atrophiques.
